# EUROPEAN PATENT APPLICATION

(11) **EP 2 420 220 A1**
(43) Date of publication of application: **22.02.2012**
(21) Application number: 10764522.8
(22) Date of filing: 16.04.2010
(51) Int. Cl.: A61K 8/39, A61K 8/34, A61K 8/86, A61Q 5/06

(54) **HAIRSTYLING COSMETIC**

(30) Priority: 16.04.2009 JP 2009099939
(71) Applicant: Shiseido Company, Ltd., Chuo-ku Tokyo 104-8010 (JP)
(72) Inventor: SHIMIZU Hideki, Yokohama-shi Kanagawa 224-8558 (JP); KURASHIMA Takumi, Yokohama-shi Kanagawa 224-8558 (JP)
(74) Representative: Henkel, Breuer & Partner
(86) International application number: PCT/JP2010/056821
(87) International publication number: WO 2010/119940

(57) **Abstract**

The present invention provides a hairstyling cosmetic that applies easily; fits smoothly to hair; thickens thus has a superior hairstyling capacity immediately after application; and has suitable setting capacity, re-hairstyling capacity, and non-stickiness because upon completion of hairstyling, entire solvent volatilizes and a hairstyling component precipitates.

The hairstyling cosmetic of the present invention is **characterized by** comprising:

(a) one or more block-type alkylene oxide derivative(s) selected from formulae (1), (II), and (III), which is solid at room temperature (25°C) and insoluble in water but soluble in lower alcohols to become transparent; and
(b) a solvent containing water and a lower alcohol,
wherein the component (a) dissolves in component (b),

and as the lower alcohol preferentially volatilizes after application, component (a) is admixed with the solvent with a changed composition, and then substantially entire solvent volatilizes:

R¹O-[(AO)ₚ(EO)_{q}(AO)ᵣ]-R² (I)

R¹O-[(EO)ₚ(AO)_{q}(EO)ᵣ]-R² (II)

R¹O-[(EO)ₚ(AO)_{q}R³(AO)ₛ(EO)ᵣ]-R² (III)

wherein AO is an oxyalkylene group having 3 to 4 carbon atoms, EO is an oxyethylene group, p, q, r, and s are the average addition mole numbers, 1≦p+r≦90, and 1≦q+s≦90, the percentage of the oxyethylene groups with respect to the sum of the oxyalkylene groups having 3 to 4 carbon atoms and the oxyethylene groups is 20 to 80 mass %; each of R¹ and R² is a hydrocarbon group having 1 to 4 carbon atoms or a hydrogen atom and they can be either the same or different; and R³ is a linear or branched hydrocarbon group having 1 to 36 carbon atoms.

## Description

### RELATED APPLICATIONS

This application claims the priority of Japanese Patent Application No. 2009-99939 filed on April 16, 2009, which is incorporated herein by reference.

### FIELD OF THE INVENTION

The present invention relates to a hairstyling cosmetic, and in particular, relates to a hairstyling cosmetic applying easily, thickening thus having a superior hairstyling effect immediately after application, fitting smoothly to hair, and having excellent re-hairstyling property and non-stickiness upon completion of hairstyling.

### BACKGROUND OF THE INVENTION

Hairstyling cosmetics are products that are used to style the hair and maintain the styled hair as well as to hydrate the hair. Such hairstyling cosmetics are sold in response to various usage patterns and preferences of consumers. Examples of hairstyling cosmetics include various products such as hair foam, hair spray, hair gel, hair wax, pomade, and hair cream.
For example, liquid form hairstyling cosmetics, in which an addition product of polyalcohol and polyalkylene glycol is blended, are disclosed (Patent literatures 1 and 2). Further, gel form, spray form, and foam form hairstyling cosmetics, in which a resin such as vinyl, acryl, or urethane is blended, are disclosed (Patent literatures 3 and 4). Furthermore, wax form hairstyling cosmetics, in which an emulsified solid wax is blended, are disclosed (Patent literatures 5 and 6).

Patent literature 1: Japanese unexamined patent publication No. S50-126841
Patent literature 2: Japanese unexamined patent publication No. S56-83414
Patent literature 3: Japanese unexamined patent publication No. H6-135823
Patent literature 4: Japanese unexamined patent publication No. 2003-171236
Patent literature 5: Japanese unexamined patent publication No. H10-45546
Patent literature 6: Japanese unexamined patent publication No. 2001-288041

### DISCLOSURE OF THE INVENTION

### PROBLEM TO BE SOLVED BY THE INVENTION

However, the conventional hairstyling cosmetics such as wax are extremely superior in terms of hairstyling capacity and re-hairstyling property because of their high viscosity; however, they do not smoothly fit to hair, thus, it is difficult to apply uniformly, as well as, they have sticky feeling after hairstyling and some attract dust to the hair.
On the other hand, the conventional hairstyling cosmetics, such as those in a liquid form, a gel form, and a spray form, fit smoothly to hair and can be applied the whole hair uniformly because they are aqueous and do not have a high viscosity; however, they have a drawback in that they are difficult to exhibit hairstyling capacity until volatile components such as water and alcohols have volatilized and dried after application.
The present invention has been carried out in view of the conventional techniques and the problem to be solved is to provide a hairstyling cosmetic that has a hairstyling effect from immediately after application, and does not have stickiness and has suitable setting capacity and excellent re-hairstyling property upon completion of hairstyling, even if an aqueous base, such as that in a liquid form, a gel form, or a spray form, which allows uniform application, is used.

### MEANS TO SOLVE THE PROBLEM

The present inventors have conducted dedicated research to solve the problem, and as a result, have discovered: by blending a specific block-type alkylene oxide derivative which is insoluble in water but soluble in lower alcohols to become transparent as a hairstyling component, it is possible to produce a hairstyling cosmetic which can fit smoothly to hair as well as can be applied uniformly upon application; moreover, which is thickened and can exert a strong hairstyling capacity after application, because a lower alcohol which has a higher volatility than water volatilizes first and the hairstyling components are admixed into the solvent with a changed composition (with a reduced amount of the lower alcohol); which can maintain arrangement capacity and re-hairstyling property, because it has a property to not solidify excessively upon completion of hairstyling; and furthermore, which is not sticky, and thereby completed the present invention.

That is, the hairstyling cosmetic of the present invention is characterized by comprising:
(a) one or more block-type alkylene oxide derivative(s) selected from formulae (I), (II), and (III), which is solid at room temperature (25°C) and insoluble in water but soluble in lower alcohols to become transparent; and
(b) a solvent containing water and a lower alcohol,
wherein the component (a) dissolves in component (b),
and as the lower alcohol preferentially volatilizes after application, component (a) is admixed with the solvent with a changed composition, and then substantially entire solvent volatilizes.

R¹O-[(AO)ₚ(EO)_{q}(AO)_{r]}-R² (I)

R¹O-[(EO)ₚ(AO)_{q}(EO)ᵣ]-R² (II)

R¹O-[(EO)ₚ(AO)_{q}R³(AO)ₛ(EO)ᵣ]-R² (III)

(In the above-described formula, AO is an oxyalkylene group having 3 to 4 carbon atoms, EO is an oxyethylene group, p, q, r, and s are the average addition mole numbers, 1 ≦p+r≦ 90, and 1 ≦ q+s≦ 90, the percentage of the oxyethylene groups with respect to the sum of the oxyalkylene groups having 3 to 4 carbon atoms and the oxyethylene groups is 20 to 80 mass %; each of R¹ and R² is a hydrocarbon group having 1 to 4 carbon atoms or a hydrogen atom and they can be either the same or different; and R³ is a linear or branched hydrocarbon group having 1 to 36 carbon atoms.)
In the hairstyling cosmetic, it is preferable that the blending quantity of component (a) is 1 to 50 mass %.
In the hairstyling cosmetic, it is preferable that the blending quantity of the water is 60 mass % or less and the blending quantity of the lower alcohol is 40 mass % or more in component (b).

### EFFECT OF THE INVENTION

According to the present invention, a hairstyling cosmetic which can fit smoothly to hair as well as can be applied uniformly upon application; which has a hairstyling capacity from immediately after application; and which has non-stickiness, suitable setting capacity, and excellent re-hairstyling capacity after hairstyling, can be obtained by blending a specific block-type alkylene oxide derivative which is insoluble in water but soluble in lower alcohols to become transparent as a hairstyling component.

### BEST MODE FOR CARRYING OUT THE INVENTION

A preferable mode for carrying out the present invention is described in the following. However, the present invention is not limited by the mode.

### (a) Block-type alkylene oxide derivative

A block-type alkylene oxide derivative(s) contained in the hairstyling cosmetic of the present invention is one or more compounds selected from the below-described formulae (I), (II), and (III) and is solid at room temperature (25°C) and insoluble in water but soluble in lower alcohols to become transparent.

R¹O-[(AO)ₚ(EO)_{q}(AO)ᵣ-R² (I)

R¹O-[(EO)ₚ(AO)_{q}(EO)r]-R² (II)

R¹O-[(EO)ₚ(AO)_{q}R³(AO)ₛ(EO)ᵣ-R² (III)

In the above-described formulae (I), (II), and (III), AO is an oxyalkylene group having 3 to 4 carbon atoms, and the specific examples include an oxypropylene group, an oxybutylene group, an oxyisobutylene group, an oxytrimethylene group, and an oxytetramethylene group. Preferably AO is an oxypropylene group or an oxybutylene group. EO is an oxyethylene group.
p, q, r, and s are the average addition mole numbers, 1≦p+r≦90, nd 1≦q+s≦90. en the number of the oxyalkylene groups having 3 to 4 carbon atoms or the number of the oxyethylene groups is zero, the smoothness decreases. When it exceeds 90, the stickiness may increase when used, thus the arrangement tends to be difficult.
In addition, the percentage of the oxyethylene groups with respect to the sum of the oxyalkylene groups having 3 to 4 carbon atoms and the oxyethylene groups is 20 to 80 mass % and preferably 40 to 70 mass %. When the percentage of the oxyethylene group is less than 20 mass %, the smoothness tends to poor. When it exceeds 80 mass %, the stickiness tends to be caused.

Each of R¹ and R² is a hydrocarbon group having 1 to 4 carbon atoms or a hydrogen atom. Examples of hydrocarbon groups include a methyl group, an ethyl group, an n-propyl group, an isopropyl group, an n-butyl group, a sec-butyl group, and a tert-butyl group. Preferably it is a methyl group or an ethyl group. In the case of a hydrocarbon group having 5 carbon atoms or more, the moist feeling tends to be lowered because of decreased hydrophilicity.
For R¹ and R², the same group may be used, respectively, a hydrocarbon group having 1 to 4 carbon atoms and a hydrogen atom may be mixed, or different hydrocarbon groups having 1 to 4 carbon atoms may be mixed. More preferably R¹ and R² are identical or different hydrocarbon groups having 1 to 4 carbon atoms.
R³ is a linear or branched hydrocarbon group having 1 to 36 carbon atoms.

The specific examples of the alkylene oxide derivatives of the present invention include POE(14)POP(7) dimethyl ether, POE(10)POP(10) dimethyl ether, POE(6)POP(14) dimethyl ether, POE(15)POP(5) dimethyl ether, POE(25)POP(25) dimethyl ether, POE(7)POP(12) dimethyl ether, POE(22)POP(40) dimethyl ether, POE(35)POP(40) dimethyl ether, POE(50)POP(40) dimethyl ether, POE(55)POP(30) dimethyl ether, POE(30)POP(34) dimethyl ether, POE(25)POP(30) dimethyl ether, POE(27)POP(14) dimethyl ether, POE(55)POP(28) dimethyl ether, POE(36)POP(41) dimethyl ether, POE(14)POB(7) dimethyl ether, POE(10)POP(10) diethyl ether, POE(10)POP(10) dipropyl ether, POE(10)POP(10) dibutyl ether, POE(52)POB(32) dimethyl ether, POE(64)POB(32) dimethyl ether, POE(34)POB(14) dimethyl ether, and POE(35)POB(32) dimethyl ether.
The above-described POE, POP, and POB are the abbreviations of polyoxyethylene, polyoxypropylene, and polyoxybutylene, respectively. Hereinafter, they may be abbreviated as such.

The blending quantity of the block-type alkylene oxide derivative in the hairstyling cosmetic of the present invention is preferably 1 to 50 mass % and more preferably 2 to 20 mass %. When the blending quantity is less than 1 mass %, the manifestation of the blending effect may not be satisfactory. When it exceeds 50 mass %, the stickiness may be caused.
The block-type alkylene oxide derivative of the present invention can be prepared according to publicly known methods. For example, they can be obtained by the addition polymerization of ethylene oxide and alkylene oxide containing 3 to 4 carbon atoms with a compound containing hydroxyl groups and the subsequent ether reaction with halogenated alkyl compound in the presence of an alkaline catalyst.

### (b) Solvent containing water and a lower alcohol

A solvent of the hairstyling cosmetic of the present invention contains water and a lower alcohol.

### Water

Water used in the present invention is not limited in particular, and the specific examples include ion-exchanged water, purified water, and tap water.
The blending quantity of the water is preferably 60 mass % or less with respect to the total amount of the hairstyling cosmetic and particular preferably 50 mass % or less. When the blending quantity exceeds 60 mass %, the block-type alkylene oxide derivative may separate, thus, the hairstyling effect tends not to be sufficient.

### Lower alcohol

As lower alcohols used in the present invention, monovalent aliphatic alcohols having 1 to 4 carbon atoms can be used, and the specific examples include ethanol, propanol, isopropanol, isobutyl alcohol, and t-butyl alcohol. Preferably ethanol can be used.
The blending quantity of the lower alcohol is preferably 40 mass % or more with respect to the total amount of the hairstyling cosmetic and particular preferably 50 mass % or more. When the blending quantity is less than 40 mass %, the block-type alkylene oxide derivative may separate, thus, the hairstyling effect tends not to be sufficient, as well as, stickiness may appear.

The hairstyling cosmetic of the present invention is one that a specific block-type alkylene oxide derivative, that is, component (a), is completely dissolved in a solvent containing water and a lower alcohol, that is, component (b). Therefore, when the hairstyling cosmetic of the present invention is applied to hair, it can fit smoothly to hair as well as can be applied uniformly. In addition, it has a superior hairstyling capacity from immediately after application because the lower alcohol preferentially volatilizes after application and component (a) is admixed with the solvent with a changed composition.

The hairstyling cosmetic of the present invention can exert hairstyling capacity and re-hairstyling property to maintain the hairstyle by volatilization of substantially entire solvent and attachment of (a) the block-type alkylene oxide derivative which is the hairstyling component on the hair at a precipitated state after hairstyling.
For the hairstyling cosmetic of the present invention, it is desirable to adjust the blending quantity and ratio of water and a lower alcohol, that is, component (b), so that volatilization time of the entire solvent under normal use would be within 20 minutes, preferably within 10 minutes. When the duration until the entire solvent volatilizes is too long, hairstyling may become difficult; however, there are methods which can hairstyle by forcibly drying with a hair dryer etc, thus, it is not particularly limited and is suitably adjustable.

In addition to the above-described essential components, the components normally used in hairstyling cosmetics can be blended in the hairstyling cosmetic of the present invention. Examples of such components include moisturizer, surfactant, UV absorber, perfume, antioxidant agent, antiseptic agent, fungicide, extender pigment, coloring pigment, and alcohol.
Moreover, in the present invention, by blending a specific block-type alkylene oxide derivative as a hairstyling component, a hairstyling cosmetics with suitable setting capacity and re-hairstyling property, fitting more smoothly to hair than hairstyling cosmetics using conventional hairstyling components, such as, wax and film-forming polymers, as well as maintaining the hairstyling capacity upon application but does not have stickiness upon completion of hairstyling can be obtained. However, it is also possible to use conventional hairstyling components, such as, wax and film-forming polymers in combination as long as the effect of the present invention is not impaired.

The hairstyling cosmetic of the present invention can be in any form, and the examples include solubilized form, emulsion form, dispersed powder form, oil-water double-layer form, and oil-water-powder triple-layer form. Examples of prefer embodiments of the hairstyling cosmetic of the present invention include aerosol-type hair spray, pump-type hair spray, foam form aerosol, hair mist, setting lotion, hairstyling gel, and hair liquid.

### EXAMPLES

Hereinafter, the present invention will be further described in the following examples. However, the present invention is not limited by these examples. The blending quantity is expressed in mass % unless otherwise noted. Prior to illustrating the examples, the rating criteria and evaluation methods used in the present invention will be described.

### Evaluation (1): Smooth fitting

0.5 g of the sample was applied to one bunch of black virgin hair (20 cm length, 4 g in mass) and fitted to the hair by finger, and then sensory testing was performed by 10 female professional panelists to evaluate the smooth fitting. The rating criteria were as follows.

### Rating criteria

5 points: smooth to fit
4 points: slightly smooth to fit
3 points: ordinary
2 points: slightly hard to fit
1 point: hard to fit

### Evaluation (2): Hairstyling capacity (arrangement capacity) upon application

0.5 g of the sample was applied to one bunch of black virgin hair (20 cm length, 2 g in mass), and then sensory testing was performed by 10 female professional panelists to evaluate the easiness of arrangement when picked and twisted, for the hair bundle immediately after the sample being uniformly spread. The rating criteria were as follows.

### Rating criteria

5 points: very easy to arrange
4 points: slightly easy to arrange
3 points: ordinary
2 points: slightly difficult to arrange
1 point: cannot arrange

### Evaluation (3): Hair fixing capacity (setting capacity) upon completion of hairstyling

0.2 g of the sample was applied to black virgin hair (10 cm length, 0.5 g in mass) and fitted to the hair using a comb, and the hair was made straight, and then dried at room temperature for 20 minutes, and 5 strands of hairs was prepared for each sample. The maximum stress when 5 mm of this strand was pressed was measured using a rheometer (RTC-2002DD (manufactured by Rheotec Messtecbnik GmbH)). The evaluation methods were as follows.

### Evaluation method

A: Maximum stress value is 200g weight or more.
B: Maximum stress value is 150g weight or more and less than 200g weight.
C: Maximum stress value is 100g weight or more and less than 150g weight.
D: Maximum stress value is 50g weight or more and less than 100g weight.
E: Maximum stress value is less than 50g weight.

### Evaluation (4): Re-hairstyling capacity (arrangement capacity) upon completion of hairstyling

After evaluation (2) was performed, the hair bundle was dried at room temperature for 20 minutes, and then sensory testing was performed by 10 female professional panelists to evaluate the easiness of arrangement when picked and twisted again. The rating criteria were as follows.

### Rating criteria

5 points: very easy to arrange
4 points: slightly easy to arrange
3 points: ordinary
2 points: slightly difficult to arrange
1 point: cannot arrange

### Evaluation (5): Absence of stickiness upon completion of hairstyling

Sensory testing was performed by 10 female professional panelists to evaluate the sticky feeling of the hair when the evaluation method (4) was performed. The rating criteria were as follows.

### Rating criteria

5 points: No stickiness is sensed at all.
4 points: No stickiness is slightly sensed.
3 points: Neither the presence nor absence of stickiness applies.
2 points: Stickiness is slightly sensed.
1 point: Stickiness is considerably sensed.

### Evaluation (6): Re-hairstyling capacity (arrangement capacity) 2 hours after application

After evaluation (2) was performed, the hair bundle was let stand for 2 hours, and then sensory testing was performed by 10 female professional panelists to evaluate the easiness of arrangement when picked and twisted again. The rating criteria were as follows.

### Rating criteria

5 points: very easy to arrange
4 points: slightly easy to arrange
3 points: ordinary
2 points: slightly difficult to arrange
1 point: cannot arrange

### Evaluation (7): Solubility

After the preparation, the solubility of each sample was visually observed. The evaluation methods were as follows.

### Evaluation method

A: transparent and uniformly dispersed
B: uniformly dispersed but milky
C: milky and non-uniformly dispersed

First, the present inventors prepared each hairstyling cosmetic, by the normal method, comprising various components as a hairstyling component with the blending composition shown in Table 1.
Then, each sample was evaluated for the evaluation item (3) in the above-described evaluation method. Also, the professional panelists evaluated each sample for the evaluation items (1), (2), and (4) to (6) according to the above-mentioned rating criteria, and the total score was evaluated in the following evaluation method. The result is shown in Table 1.

### Evaluation method

A: Total points are 40 or more.
B: Total points are 30 or more and less than 40.
C: Total points are 20 or more and less than 30.
D: Total points are less than 20.

**[Table 1]**

| | Test Example | | | | | | |
|---|---|---|---|---|---|---|---|
| | 1-1 | 1-2 | 1-3 | 1-4 | 1-5 | 1-6 | 1-7 |
| POE(34)·POB(14) dimethyl ether | 20 | - | - | - | - | - | - |
| Polyvinylpyrrolidone/vinyl acetate copolymer | - | 20 | - | - | - | - | - |
| Acrylic resin alkanolamine | - | - | 20 | - | - | - | - |
| Amphoteric urethane resin (20 mass % aqueous dispersion) | - | - | - | 20 | - | - | - |
| Silylated urethane resin (40 mass % aqueous dispersion) | - | - | - | - | 20 | - | - |
| Beeswax | - | - | - | - | - | 20 | - |
| Microcrystalline wax | - | - | - | - | - | - | 20 |
| POE cetyl ether(25,E.O.) | - | - | - | - | - | 5 | 5 |
| Ethanol | 50 | 50 | 50 | 50 | 50 | 0 | 0 |
| Ion-exchanged water | 30 | 30 | 30 | 30 | 30 | 75 | 75 |
| (1) Smooth fitting | A | A | A | A | A | B | C |
| (2) Hairstyling capacity (arrangement capacity) upon application | A | B | B | B | C | A | A |
| (3) Hair fixing capacity (setting capacity) upon completion of hairstyling | B | B | A | A | A | C | C |
| (4) Re-hairstyling capacity (arrangement capacity) upon completion of hairstyling | A | C | C | B | C | A | A |
| (5) Absence of stickiness upon completion of hairstyling | A | C | C | C | B | C | B |
| (6) Re-hairstyling capacity (arrangement capacity) 2 hours after applied | A | C | C | B | C | A | A |

According to Table 1, Test Example 1-1, in which alkylene oxide derivative was blended as a hairstyling component, was a hairstyling cosmetic which fitted smoothly to hair and was excellent in hairstyling capacity upon application. Further, it had a suitable fixing capacity (setting capacity) upon completion of hairstyling because the solvent in the hairstyling cosmetic volatilized by then. In spite of this, it had no stickiness, and re-hairstyling could be carried out to the hair bundle 2 hours later.
On the other hand, Test Example 1-2, in which polyvinylpyrrolidone/vinyl acetate copolymer was blended as a hairstyling component, fitted smoothly to the hair; however, it was a slightly poor in hairstyling capacity upon application and poor in non-stickiness as well as re-hairstyling property.
Test Example 1-3, in which acrylic resin alkanolamine was blended as a hairstyling component, fitted smoothly to hair and was excellent in setting capacity upon completion of hairstyling; however, it was a slightly poor in hairstyling capacity upon application, and re-hairstyling was not possible and it had also stickiness once dried and the resin solidifies.
Test Example 1-4, in which amphoteric urethane resin was blended as a hairstyling component, fitted smoothly to hair and was excellent in setting capacity upon completion of hairstyling; however, it was slightly poor in hairstyling capacity upon application as well as re-hairstyling capacity, and also had stickiness.
Test Example 1-5, in which silylated urethane resin was blended as a hairstyling component, fitted smoothly to hair and was excellent in setting capacity upon completion of hairstyling; however, the hairstyling capacity upon application was not sufficient, and re-hairstyling was not possible and it had also stickiness once dried and the resin solidifies.
Test Example 1-6, in which beeswax was blended as a hairstyling component, was excellent in arrangement capacity as well as re-hairstyling property to hair upon application; however, it was slightly poor in fitting to hair and moreover was poor in setting capacity to hair upon completion of hairstyling, and was also extremely sticky.
Test Example 1-7, in which microcrystalline wax was blended as a hairstyling component, was excellent in arrangement capacity to hair upon application as well as re-hairstyling property; however, stickiness was observed upon completion of hairstyling and fitted hardly to hair and was poor in setting capacity.

In conclusion, it was found that when an alkylene oxide derivative is blended as a hairstyling component, a hairstyling cosmetic, which fits smoothly to hair, has a hairstyling capacity (arrangement capacity) upon application and also has suitable setting capacity as well as superior re-hairstyling capacity (arrangement capacity) even upon completion of hairstyling, has less stickiness, as well as allows re-hairstyling over time, as compared to those hairstyling cosmetics blended with conventional hairstyling components, can be prepared.

The present inventors studied alkylene oxide derivatives blended as a hairstyling component.
First, regarding the alkylene oxide derivatives of compounds 1 to 12 shown below in Table 2, the states at room temperature (25°C) and 60°C as well as the solubilities in water and ethanol were confirmed. Those which were completely soluble was shown in O, those which were observed with some left without being dissolved was shown in A, and those which did not dissolve at all was shown in × as for the evaluation. The result is shown in Table 2.

**[Table 2]**

| | | State(room temparature) | State (60°C) | Solubility in water | Solubility in ethanol |
|---|---|---|---|---|---|
| Compound 1 | Formula (I); POE(34)·POB(14) dimethyl ether(R¹=R²=CH₃) | Solid | Liquid | × | ○ |
| Compound 2 | Formula (I); POE(45)·POB(28) dimethyl ether(R¹=R²=CH₃) | Solid | Liquid | × | ○ |
| Compound 3 | Formula (I); OE(52)·POB(32) dimethyl ether(R¹=R²=CH₃) | Solid | Liquid | × | ○ |
| Compound 4 | Formula (I); POE(64)·POB(32) dimethyl ether(R¹=R²=CH₃) | Solid | Liquid | × | ○ |
| Compound 5 | Formula (II); POE(20)·POB(28) dimethyl ether(R¹=R²=CH₃) | Solid | Liquid | × | ○ |
| Compound 6 | Formula (III); POE(52)·POB(25) dimethyl ether(R³; hydrocarbon group having 36 carbon atoms) (R¹=R²=CH₃ | Solid | Liquid | × | ○ |
| Compound 7 | Formula (III); POE(34)·POB(25) dimethyl ether(R³; hydrocarbon group having 36 carbon atoms) (R¹=R²=CH₃) | Solid | Liquid | × | ○ |
| Compound 8 | Formula (I); POE(52)·POB(32) dimethyl ether(R¹=R²=H) | Solid | Liquid | × | ○ |
| Compound 9 | Formula (I); POE(23)·PO(32) dimethyl ether(R¹=R²=CH₃) | Liquid | Liquid | × | ○ |
| Compound 10 | Formula (II); POE(73)·POB(11) dimethyl ether (R¹= R²=CH₃) | Solid | Liquid | ○ | ○ |
| Compound 11 | Polyethyleneglycol (molecular weight: 4000) (biterminal: OH) | Solid | Liquid | ○ | × |
| Compound 12 | POE(36)·POP(41) dimethyl ether(random copolymerization) (R¹=R²=CH₃) | Liquid | Liquid | Δ | ○ |

The present inventors prepared each hairstyling cosmetic (transparent hair cosmetic), by the following production method, using each above-described alkylene oxide derivative with the blending composition shown in Tables 3 and 4.

### Hair cosmetic production method

Each of compounds 1 to 12 is heated and melted and then dissolved into ethanol. Subsequently, ion exchanged water was added to this, stirred and mixed, and a liquid form hairstyling agent was obtained.

The professional panelists evaluated each sample for the evaluation items (1), (2), and (4) according to the above-mentioned rating criteria, and the total score was evaluated in the following evaluation method. The results are shown in Tables 3 and 4.

### Evaluation method

A: Total points are 40 or more.
B: Total points are 30 or more and less than 40.
C: Total points are 20 or more and less than 30.
D: Total points are less than 20.

**[Table 3]**

| | Test Example | | | | | | | |
|---|---|---|---|---|---|---|---|---|
| | 2-1 | 2-2 | 2-3 | 2-4 | 2-5 | 2-6 | 2-7 | 2-8 |
| Ion-exchanged water | 30 | 30 | 30 | 30 | 30 | 30 | 30 | 30 |
| Ethanol | 50 | 50 | 50 | 50 | 50 | 50 | 50 | 50 |
| Compound 1 | 20 | - | - | - | - | - | - | - |
| Compound 2 | - | 20 | - | - | - | - | - | - |
| Compound 3 | - | - | 20 | - | - | - | - | - |
| Compound 4 | - | - | - | 20 | - | - | - | - |
| Compound 5 | - | - | - | - | 20 | - | - | - |
| Compound 6 | - | - | - | - | - | 20 | - | - |
| Compound 7 | - | - | - | - | - | - | 20 | - |
| Compound 8 | - | - | - | - | - | - | - | 20 |
| (1) Smooth fitting | A | A | A | A | A | A | A | A |
| (2) Hairstyling capacity (arrangement capacity) upon application | A | A | A | A | A | A | A | A |
| (4) Re-hairstyling capacity (arrangement capacity) upon completion of hairstyling | A | A | A | A | A | A | A | A |

**[Table 4]**

| | Test Example | | | |
|---|---|---|---|---|
| | 2-9 | 2-10 | 2-11 | 2-12 |
| Ion-exchanged water | 30 | 30 | 30 | 30 |
| Ethanol | 50 | 50 | 50 | 50 |
| Compound 9 | 20 | - | - | - |
| Compound 10 | - | 20 | - | - |
| Compound 11 | - | - | 20 | - |
| Compound 12 | - | - | - | 20 |
| (1) Smooth fitting | A | A | B | A |
| (2) Hairstyling capacity (arrangement capacity) upon application | C | C | C | C |
| (4) Re-hairstyling capacity (arrangement capacity) upon completion of hairstyling | C | A | A | C |

According to Table 3, the hairstyling cosmetics of Test Examples 2-1 to 2-8, in which block-type alkylene oxide derivative (one or more compounds selected from the formula (1), (II), or (III)) according to the present invention which are solid at room temperature and insoluble in water but soluble in lower alcohols to become transparent was blended, fitted smoothly to hair and were excellent in hairstyling capacity upon application as well as re-hairstyling capacity (arrangement capacity) upon completion of hairstyling.
However, Test Example 2-9, in which Compound 9, which is a block-type alkylene oxide derivative represented by the formula (I) but liquid at room temperature was blended, fitted smoothly to hair; however, it was poor in hairstyling capacity upon application and re-hairstyling capacity (arrangement capacity) upon completion of hairstyling.
Moreover, Test Example 2-10, in which Compound 10, which is about 87 mass % of the ratio of the number of oxyethylene groups to the sum of the numbers of oxyalkylene groups having 3 to 4 carbon atoms and oxyethylene groups in the block-type alkylene oxide derivative of formula (II) and soluble in water and lower alcohols to become transparent was blended, fitted smoothly to hair and was excellent in re-hairstyling capacity (arrangement capacity) upon completion of hairstyling; however, it was poor in hairstyling capacity upon application.
In addition, Test Example 2-11, in which polyethylene glycol with a molecular weight of 4000 which is solid at room temperature and soluble in water but insoluble in lower alcohols (Compound 11) was blended, was excellent in re-hairstyling capacity (arrangement capacity) upon completion of hairstyling; however, it was slightly poor in fitting to hair and poor in hairstyling capacity upon application.
Furthermore, Test Example 2-12, in which random-type alkylene oxide derivative which is liquid at room temperature and also was slightly poor in solubility in water (Compound 12) was blended, fitted smoothly to hair; however, it was poor in hairstyling capacity upon application as well as re-hairstyling capacity (arrangement capacity) upon completion of hairstyling.

From the above, it was clarified that the hairstyling cosmetic, in which (a) (a) one or more block-type alkylene oxide derivative(s) selected from formulae (I), (II), and (III), which is solid at room temperature (25°C) and insoluble in water but soluble in lower alcohols to become transparent, as a hairstyling component, as well as (b) a solvent containing water and a lower alcohol were blended, fitted smoothly to hair and had hairstyling capacity from immediately after application. Such hairstyling cosmetic of the present invention had suitable setting capacity, re-hairstyling capacity, and non-stickiness because after completion of hairstyling, entire solvent volatilizes and a hairstyling component precipitates on hair.

Next, the present inventors prepared each hairstyling cosmetic, by the above-described production method, comprising specific (a) block-type alkylene oxide derivative of the present invention as well as (b) various blending quantities of water and a lower alcohol and various kinds of a lower alcohol, with the blending composition shown in Table 5. Then, each sample was evaluated for the evaluation items (1) to (6) along with the above-described Table 1 and the evaluation item (7) in the above-described evaluation method. The result is shown in Table 5.

**[Table 5]**

| | | Test Example | | | | | | | |
|---|---|---|---|---|---|---|---|---|---|
| | | 3-1 | 3-2 | 3-3 | 2-3 | 3-4 | 3-5 | 3-6 | 3-7 |
| (a) | Compound 3 | 20 | 20 | 20 | 20 | 20 | 20 | 20 | 20 |
| (b) | Ethanol | 20 | 30 | 40 | 50 | - | - | - | - |
| | Propanol | - | - | - | - | 20 | 30 | 40 | 50 |
| | Ion-exchanged water | 60 | 50 | 40 | 30 | 60 | 50 | 40 | 30 |
| (1) Smooth fitting | | D | D | B | A | D | B | A | A |
| (2) Hairstyling capacity (arrangement capacity) upon application | | D | D | B | A | D | B | A | A |
| (3) Hair fixing capacity (setting capacity) upon completion of hairstyling | | C | C | B | B | C | B | B | B |
| (4) Re-hairstyling capacity (arrangement capacity) upon completion of hairstyling | | C | C | A | A | C | A | A | A |
| (5) Absence of stickiness upon completion of hairstyling | | C | C | A | A | C | A | A | A |
| (6) Re-hairstyling capacity (arrangement capacity) 2 hours after applied | | C | C | A | A | C | A | A | A |
| (7) Solubility | | C | C | B | A | C | B | A | A |

According to Table 5, in the case that ethanol was blended as a lower alcohol, Test Examples 3-1 and 3-2, in which the blending quantity of ethanol is small and the blending quantity of water is large, were poor in all evaluations and could not achieve improvement of the hairstyling capacity along with preferential ethanol volatilization. Moreover, because the solubility of component (a) is poor, the component (a) caused milky appearance and did not disperse uniformly.
On the other hand, as shown in Test Examples 3-3 and 2-3, as the blending quantity of ethanol was increased, uniform dispersion could be made and a hairstyling cosmetic with not only the hairstyling capacity upon application but also the hairstyling capacity improving along with preferential ethanol volatilization was obtained.
In addition, in the case that propanol was blended as a lower alcohol, Test Example 3-4, in which the blending quantity of propanol was small and the blending quantity of water was large, could not achieve improvement of the hairstyling capacity along with preferential propanol volatilization. Moreover, because the solubility of component (a) is poor, the component (a) caused milky appearance cloudy and did not disperse uniformly.
On the other hand, as shown in Test Examples 3-5 to 3-7, as the blending quantity of propanol was increased, uniform dispersion could be made and a hairstyling cosmetic with not only the hairstyling capacity upon application but also the hairstyling capacity improving along with preferential propanol volatilization was obtained.

Accordingly, in the hairstyling cosmetic of the present invention, it is necessary that component (a) dissolves in component (b). Further, in the hairstyling cosmetic, in which a specific block-type alkylene oxide derivative (a) is blended as a hairstyling component, it is preferred to blend 40 mass % or more of a lower alcohol and 60 mass % or less of water in the total amount of the cosmetic. Considering the solubility of component (a), smooth fitting, and hairstyling capacity upon application, it is particularly preferred to blend 50 mass % or more of a lower alcohol.

In the following, formulation examples of the hairstyling cosmetic of the present invention will be listed. However, the technical scope of the present invention is not limited by these. The obtained hairstyling cosmetics can fit smoothly to hair as well as can be applied uniformly and have a hairstyling capacity from immediately after application. The cosmetics have also non-stickiness and re-hairstyling capacity because entire solvent volatilizes upon completion of hairstyling.

### Formulation Example 1 Mist-form hairstyling agent

| | (mass %) |
|---|---|
| (1) Ion-exchanged water | remainder |
| (2) Ethanol | 50.0 |
| (3) Compound 1 | 10.0 |
| (4) Sorbitol | 3.0 |
| (5) POP(70) decaglyceryl ether | 3.0 |
| (6) Polyvinylpyrrolidone/dimethyl aminoethyl methacrylic acid copolymer diethyl sulfate | 3.0 |
| (GAFQUAT 755N (manufactured by ISP Japan Ltd.)) | |
| (7) Hydrolyzed wheat protein | 0.5 |
| (CROPEPTIDE W (manufactured by Croda Japan KK)) | |
| (8) Trimethylglycine | 0.5 |
| (9) Polyether denatured silicone | 0.5 |
| (FZ-2222 (manufactured by Dow Coming Toray Co., Ltd.)) | |
| (10) Perfume | proper quantity |

### <Production method>

Melted (3), (5), (6), (9), and (10) were added to (2) in this order and dissolved to become transparent, and the resultant was then added to an aqueous phase part in which (4), (7), and (8) were dissolved in (1). This mixture was stirred for dissolution to obtain a mist-form hairstyling agent (hair-mist) having a transparent appearance.

### Formulation Example 2 Mist-form hairstyling agent

| | (mass %) |
|---|---|
| (1) Ion-exchanged water | remainder |
| (2) Ethanol | 40.0 |
| (3) Compound 2 | 20.0 |
| (4) Trehalose | 0.5 |
| (5) POP(9) diglyceryl | 3.0 |
| (6) Methacryloyl oxyethyl carboxybetaine/alkyl methacrylatecopolymer | 3.0 |
| (YUKAFORMER R205 (manufactured by Mitsubishi Chemical Corporation)) | |
| (7) Hydroxyethylurea | 0.5 |
| (8) Stearyltrimethylammonium chloride (25% aqueous solution) | 0.5 |
| (9) Polyether denatured silicone | 0.5 |
| (SS-2802 (manufactured by Dow Coming Toray Co., Ltd.)) | |
| (10) Perfume | proper quantity |

### <Production method>

Melted (3), (5), (6), (9) and (10) were added to (2) in this order and dissolved to become transparent, and the resultant was then added to an aqueous phase part in which (4), (7), and (8) were dissolved in (1). This mixture was stirred for dissolution to obtain a mist-form hairstyling agent (hair-mist) having a transparent appearance.

### Formulation Example 3 Mist-form hairstyling agent

| | (mass %) |
|---|---|
| (1) Ion-exchanged water | remainder |
| (2) Isopropanol | 40.0 |
| (3) Compound 3 | 5.0 |
| (4) Lactic acid | 0.02 |
| (5) POP(9) diglyceryl | 3.0 |
| (6) Acrylic resin alkanolamine | 3.0 |
| (PLAS CIZE L-9909B (manufactured by GOO Chemical Co., Ltd.)) | |
| (7) Polyethyleneglycol (molecular weight: 1500) | 0.5 |
| (8) POE(3). POP(20) oligo succinate | 0.5 |
| (ESTEMOL 50 (manufactured by The Nisshin OilliO Group, Ltd.)) | |
| (9) Dicocoylethyl hydroxyethylmonium methosulfate | 0.1 |
| (DEHYQUART L80 (manufactured by Cognis Japan)) | |
| (10) Perfume | proper quantity |

### <Production method>

Melted (3), (5), (6), (8), and (10) were added to (2) in this order and dissolved to become transparent, and the resultant was then added to an aqueous phase part in which melted (7), (4), and (9) were dissolved in (1). This mixture was stirred for dissolution to obtain a mist-form hairstyling agent (hair-mist) having a transparent appearance.

### Formulation Example 4 Gel-form hairstyling agent

| | (mass %) |
|---|---|
| (1) Ion-exchanged water | remainder |
| (2) Ethanol | 50.0 |
| (3) Compound 4 | 20.0 |
| (4) Diglycerin | 5.0 |
| (5) Xylitol | 0.5 |
| (6) Hydroxypropylcellulose | 1.0 |
| (HPC-H (manufactured by NIPPON SODA CO., LTD.)) | |
| (7) Vinylpyrrolidone/N,N-dimethyl aminoethyl methacrylate/alkyl acrylate/tripropylene glycol diacrylate copolymer | 0.5 |
| (Cosquat GA467 (manufactured by OSAKA ORGANIC CHEMICAL INDUSTRY LTD.)) | |
| (8) Lactic acid | 0.3 |
| (9) Stearyltrimethylammonium chloride (25%) | 0.5 |
| (10) Polyether denatured silicone | 0.5 |
| (SH3771 (manufactured by Dow Coming Toray Co., Ltd.)) | |
| (11) Sodium poly-γ-glutamate | 0.1 |
| (GELPROTEIN A-8001 (manufactured by Idemitsu TechnofineCo., Ltd.)) | |
| (12) Peppermint oil | 0.1 |

### <Production method>

Melted (3), (6), (7), (10), and (12) were added to (2) and were uniformly dispersed, which was used as an alcohol part. At the same time, (4), (5), (8), (9), and (11) were added to (1) in this order and dissolved to become transparent. This mixture was added to the alcohol part and uninformed with a homomixer to obtain a hairstyling agent of a transparent gel form.

### Formulation Example 5 Gel-form hairstyling agent

| | (mass %) |
|---|---|
| (1) Ion-exchanged water | remainder |
| (2) Ethanol | 50.0 |
| (3) Compound 5 | 30.0 |
| (4) POE(5)· POP(65) pentaerythritol ester | 2.0 |
| (5) Polyethyleneglycol (molecular weight: 6000) | 2.0 |
| (6) Carboxy vinyl polymer | 0.2 |
| (Hiviswako 105 (manufactured by Wako Pure Chemical Industries, Ltd.)) | |
| (7) Acrylate/alkyl acrylate (C 10-C30) copolymer | 0.2 |
| (PEMULEN TR-2 (manufactured by B.F.Goodrich Chemical Company)) | |
| (8) 2-amino methyl propanol | 0.3 |
| (9) POE(24)· POP(13) decyl tetradecyl ether | 0.5 |
| (10) Sodium dilauramidoglutamide lysine | 0.3 |
| (11) Rosa roxburghii fruit extract | 0.1 |
| (12) Perfume | 0.1 |

### <Production method>

(6) and (7) were added to (1) and dispersed well, and then, melted (5), (10), and (11) were added thereto to prepare an aqueous phase part. At the same time, melted (3), (4), (9), and (12) were added to (2) and dissolved to become transparent. This mixture was added to the aqueous phase part and further (8) was added and stirred to obtain a hairstyling agent of a transparent gel form.

### Formulation Example 6 Gel-form hairstyling agent

| | (mass %) |
|---|---|
| (1) Ion-exchanged water | remainder |
| (2) Ethanol | 50.0 |
| (3) Compound 6 | 10.0 |
| (4) Inositol | 0.4 |
| (5) POE(10) methyl glucoside | 2.0 |
| (Glucam E-10 (manufactured by The Lubrizol Corporation)) | |
| (6) Polyvinylpyrrolidone/vinyl acetate copolymer | 0.2 |
| (PVP/VA S-630 (manufactured by ISP Japan Ltd.)) | |
| (7) Dimethyl acrylamide/sodium acryloyldimethyltaurate crosspolymer | 1.0 |
| (Synthetic Example 4 in Japanese unexamined patent publication No. 2005-132782) | |
| (8) POE(50) oleyl ether | 0.5 |
| (9) Polyether denatured silicone | 0.3 |
| (FZ-2222 (manufactured by Dow Coming Toray Co., Ltd.)) | |
| (10) Pearl protein extract | 0.1 |
| (11) Perfume | 0.1 |

### <Production method>

Melted (3) and (8) and subsequently (9) and (11) were added to (2). (7) was added therein and were uniformly dispersed, which was used as an alcohol part. At the same time, (4), (5), (6), and (10) were added to (1) and dissolved. This mixture was added to the alcohol part and uniformly stirred to obtain a hairstyling agent of a transparent gel form.

### Formulation Example 7 Aerosol spray

| | (mass %) |
|---|---|
| (1) Ion-exchanged water | remainder |
| (2) Ethanol | 50.0 |
| (3) Compound 1 | 10.0 |
| (4) Acrylic resin alkanolamine | 5.0 |
| (PLAS CIZE L-9909B (manufactured by GOO Chemical Co., Ltd.)) | |
| (5) Diglycerin | 0.5 |
| (6) Perfume | 0.1 |

### <Production method>

Melted (3) and subsequently (4) and (6) were added to (2) and were stirred until became uniform, which was used as an alcohol part. At the same time, (5) was added to (1) and further, the alcohol part was added and uniformly stirred to prepare a stock solution. Further, 60% of this stock solution and 40% of dimethyl ether were filled into an aerosol can and mixed to obtain an aerosol spray.

### Formulation Example 8 Mist-form hairstyling agent

| | (mass %) |
|---|---|
| (1) Ion-exchanged water | remainder |
| (2) Ethanol | 50.0 |
| (3) Compound 7 | 5.0 |
| (4) 1,3-butylene glycol | 5.0 |
| (5) Perfume | 0.2 |
| (6) Wine extract | 0.1 |

### <Production method>

Melted (3) and subsequently (5) and (6) were added to (2) and were uniformly dispersed, which was used as an alcohol part. Then, (4) was added to (1) and dissolved to become transparent. This mixture was added to the alcohol part to obtain a hairstyling agent of a transparent mist form.

## Claims

1. A hairstyling cosmetic comprising:
(a) one or more block-type alkylene oxide derivative(s) selected from formulae (I), (II), and (III), which is solid at room temperature (25°C) and insoluble in water but soluble in lower alcohols to become transparent; and
(b) a solvent containing water and a lower alcohol,
wherein the component (a) dissolves in component (b),
and as the lower alcohol preferentially volatilizes after application, component (a) is admixed with the solvent with a changed composition, and then substantially entire solvent volatilizes:
R¹O-[(AO)ₚ(EO)_{q}(AO)ᵣ]-R² (I)
R¹O-[(EO)ₚ(AO)_{q}(EO)ᵣ]-R² (II)
R¹O-[(EO)ₚ(AO)_{q}R³(AO)ₛ(EO)ᵣ]-R² (III)
wherein AO is an oxyalkylene group having 3 to 4 carbon atoms, EO is an oxyethylene group, p, q, r, and s are the average addition mole numbers, 1 ≦p+r≦90, and 1≦q+s≦90, the percentage of the oxyethylene groups with respect to the sum of the oxyalkylene groups having 3 to 4 carbon atoms and the oxyethylene groups is 20 to 80 mass %; each of R¹and R² is a hydrocarbon group having 1 to 4 carbon atoms or a hydrogen atom and they can be either the same or different; and R³ is a linear or branched hydrocarbon group having 1 to 36 carbon atoms.

2. The hairstyling cosmetic according to claim 1, wherein the blending quantity of component (a) is 1 to 50 mass %.

3. The hairstyling cosmetic according to claim 1 or 2, wherein the blending quantity of the water is 60 mass % or less and the blending quantity of the lower alcohol is 40 mass % or more in component (b).
